Europäisches Patentamt

**European Patent Office** (11) Publication number: **0 074 713**

Office européen des brevets **A1**

(19)

(12) **EUROPEAN PATENT APPLICATION**

(21) Application number: **82304164.5**

(22) Date of filing: **06.08.82**

(51) Int. Cl.³: **C 13 K 13/00**
**C 07 C 29/10**

(30) Priority: **14.09.81 US 301728**

(43) Date of publication of application:
**23.03.83 Bulletin 83/12**

(84) Designated Contracting States:
**BE FR GB IT**

(71) Applicant: **ICI AMERICAS INC**
**Concord Pike & New Murphy Road**
**Wilmington Delaware 19897(US)**

(72) Inventor: **Kruse, Walter Frank**
**1 Woodbury Court**
**Wilmington Delaware 19805(US)**

(72) Inventor: **de Berardinis, Albert Joseph**
**113 Sherbrooke Drive**
**Wilmington Delaware 19808(US)**

(74) Representative: **Aufflick, James Neil et al,**
**Imperial Chemical Industries PLC Legal Department:**
**Patents Thames House North Millbank**
**London SW1P 4QG(GB)**

(54) **Method for concentrating mannose in aqueous glucose solutions.**

(57) Glucose is separated from an aqueous solution containing glucose and mannose by saturating said solution with sodium halide to form an insoluble sodium halide/glucose hydrate adduct which is precipitated. The mannose rich solutions are subsequently demineralized and reduced by hydrogenation to produce mannitol/sorbitol concentrates wherein the ratio of mannitol to sorbitol is at least 1/1.

EP 0 074 713 A1

$3240\backslash E.P.$

FSP 288

Method for concentrating mannose in aqueous glucose solutions

This invention relates to a method for concentrating mannose in aqueous solutions containing glucose and to a semi-continuous process for preparing high concentrations of mannose comprising the steps of epimerizing glucose to aqueous solutions containing glucose and mannose, precipating a sodium halide adduct of glucose hydrate to yield a filtrate rich in mannose, and separating the excess sodium halide from the filtrate. The glucose hydrate adduct can be recycled to the epimerization step for further conversion to mannose. The mannose rich filtrate which is produced can be hydrogenated to produce sorbitol/mannitol concentrates rich in mannitol.

It is well known that a mixture of sorbitol and mannitol in aqueous solution can be produced by catalytic hydrogenation of invert sugar, which is an approximately eqimolar mixture of glucose and fructose. Invert sugar in turn is commonly obtained by inversion of sucrose. Processes for hydrogenation of invert sugar are disclosed, for example, in US Patent Numbers 2 759 024; 3 329 729; 3 705 199; and 3 763 246. The yield of mannitol is ordinarily about 24 - 26 percent by weight based on total dry solids; this yield can be increased to about 30 percent by weight by appropriate choice of catalyst, as described in US Patent Numbers 3 705 199 and 3 363 246. Mannitol is readily recovered from aqueous solution containing both sorbitol and mannitol by fractional crystalization as described, for example, in US Patent 3 632 656. Hydrogenation of invert sugar is an attractive commercial route to the production of mannitol when the price of sucrose is low. However, recent sharp rises in the price of sucrose have indicated a need for alternate economic processes. It is also known that glucose can be isomerized under alkaline conditions to yield a mixture containing glucose, fructose and mannose. Various alkaline materials for this purpose including sodium hydroxide and calcium hydroxide are known. The proportion of glucose, fructose and mannose in the reaction mixture will vary depending on the alkaline material and the conditions used and significant quantities of mannose are not always obtained. More recently, an improved process has been described in US 4 029 878 wherein mannose is produced in enhanced yields from glucose solutions containing at least 50 percent by weight carbohydrate by epimerizing with a catalyst containing molybdenum in the hexavalent state at a temperature of at least $70^{o}C$. A solution containing an average of 29 - 34 percent by weight mannose is thereby produced. Such solutions when catalytically hydrogenated to produce a solution containing sorbitol and mannitol ultimately result in a sorbitol/mannitol blend containing about 30% mannitol. From this, mannitol is recovered by fractional crystalization. By this technique therefore for every 3 parts mannitol produced 7 parts of sorbitol results as a by-product. In the process of this invention mannose and mannitol can be recovered such that for every 1 part mannitol only 1 part or less of sorbitol is produced as a by-product.

It is therefore an object of the invention to provide a process for the concentration of mannose in glucose solutions by the formation of a relatively insoluble glucose hydrate/sodium halide adduct to precipitate out glucose from the solution.

It is another object of the invention to provide a process for the epimerization of glucose to form a solution of glucose and mannose wherein at least a portion of the glucose originates as a glucose hydrate/sodium halide adduct derived from the filtration of said adduct from a solution containing the glucose hydrate/sodium halide adduct and mannose.

These and other objects of the invention are described but not limited to the process details which follow:

According to the present invention we provide a process for concentrating mannose in an aqueous carbohydrate solution containing glucose and mannose having a carbohydrate solids content of 60 - 88% by weight which comprises the steps of:

(a)      forming an insoluble glucose hydrate/sodium halide adduct in said carbohydrate solution,

(b)      separating said insoluble adduct from said aqueous solution, and

(c)      collecting the mannose rich solution resulting therefrom.

Further according to the present invention we provide a process for producing mannitol rich concentrates from a carbohydrate solution containing glucose which comprises the steps of:

(1)      epimerizing said carbohydrate solution containing glucose to form a solution containing mannose and glucose,

(2)      forming an insoluble glucose hydrate/sodium halide adduct,

(3)      separating said insoluble adduct to form a solution rich in mannose,

(4)      demineralizing said mannose rich solution, and

(5)      hydrogenating said mannose rich solution to form mannitol.

This process for the production of mannitol also includes the step of recycling the separated glucose hydrate/sodium halide adduct separated in Step 3 back to the epimerization Step 1.

The term "glucose" refers to D-glucose and the term "mannose" refers to D-mannose throughout the specification. The term "sodium halide" refers to the iodide, bromide and most preferably the chloride salt of sodium.

The starting carbohydrate used in this invention is glucose, a polymer of glucose, or a mixture of glucose and polymers thereof. The carbohydrate also includes mannose and other sugar products in minor amounts. Glucose is the preferred carbohydrate and the description herein will be directed primarily to processes using glucose as the starting material.

The solutions containing glucose and mannose are usually derived by the epimerization of solutions containing at least about 50 percent by weight carbohydrate in water. Any conventional technique for converting glucose to solutions containing glucose and mannose is operable, however. A preferred technique as described in US 4 029 878 provides for a catalytic epimerization wherein glucose is converted to solutions of glucose and mannose at a pH range of about 3 - 5 employing catalysts comprising molybdenum in the hexavalent state such as molybdic acid, isopolymolybdic acid, heteropoly molybdic acid, and acid salts such as sodium phosphoromolybdic and silicomolybdic acid or such molybdenum compounds deposited on an anion exchange resin in which the hydroxyl ions have been replaced by molybdenum ions. This process is preferred and the teachings of US 4 029 878 are hereby incorporated by reference.

Preparation 1

An aqueous solution of 70 percent by weight glucose is prepared by adding 120 grams of glucose to distilled water in a 180 millilitre soda pop bottle at atmospheric pressure and room temperature (about $25^{\circ}$C). To this is added 30 grams of a catalyst prepared by depositing 1 gram of molybdic acid on 5 grams of Amberlyst A-26 a commercially available styrene-divinylbenzene quartenary anion exchange resin obtained from Rohm and Haas Company. The pH of the solution is adjusted to about 4. The bottle is stoppered and the solution is magnetically stirred in a constant temperature bath at $120^{\circ}$C for 30 minutes. The reaction is carried out at ambient pressure that is the pressure which develops as a result of heating the closed bottle and its contents. The bottle is then removed from constant temperature bath and allowed to cool. Water is added to dilute the solution to about 50% carbohydrate concentrate and the solution is filtered to separate catalyst. Analysis of the filtrate shows that the mixture contains about 33 percent by weight mannose.

The process of the invention is directed to further concentrating glucose and mannose primarily for the purpose of generating solutions which contain a higher concentration of mannose (mannose/glucose weight ratios of 1/1 to 2.5/1) than previously obtainable by conventional techniques and which can be hydrogenated to form solutions containing high concentrations of mannitol in the presence of equivalent or lesser amounts of sorbitol.

It is known that glucose forms an adduct with sodium chloride which precipitate from aqueous solutions containing about 8.5% sodium chloride as described in <u>Advances in Carbohydrate Chemistry</u>, N L Wolfrom, Volume 21, page 211 (1966). However, nothing has been found concerning the similar formation of salts with mannose. Furthermore, it is known that glucose can be separated from fructose by the precipitation of the glucose sodium chloride double salt from solutions containing fructose at a pH of from 7 - 9 from sugar solutions containing at least 42% carbohydrate as shown in US 3 671 316. Even though glucose can be separated from fructose, it is surprising that glucose and mannose form double salts with sufficiently different solubility since their only difference is the substitution of the hydroxyl group at the second carbon atom. It is known however that the solubility of alpha glucose hydrate is 82 grams per 100 millilitres water at $25^{\circ}C$ while solubility of mannose hydrate is 248 grams per 100 millilitres at $17^{\circ}C$.

The process of the present invention involves a separation of glucose from mannose in aqueous solutions containing from 60 - 88% and preferably 68 - 72% by weight carbohydrate solids wherein the glucose to mannose weight ratio ranges from about 9-0.25. The glucose hydrate/sodium halide adduct precipitates upon standing at 20 - $70^{\circ}C$ in solutions which are concentrated in water soluble sodium halide.

The invention is illustrated by the following Examples in which all percentages and proportions are expressed in percent or parts by weight.

<u>EXAMPLE 1</u>

A 400 part glucose epimerizate resulting from Preparation 1 containing about 55 percent by weight solid carbohydrate is heated to 60 - 70°C and mixed with 40 parts sodium chloride until completely dissolved. 80 parts water is evaporated at 60°C over a period of 3 hours. The solution is cooled slowly to room temperature under circulating air over a period of 4 hours to allow a precipitate to form. The slurry is filtered to remove 120 parts wet crystals and 211 parts filtrate containing 60% solids. The precipitate is washed with 30 parts water and collected. The dried precipitate has a glucose/mannose weight ratio of 20/1. The filtrate and wash is treated with a total of 250 parts mixed-bed ion exchange resin to remove sodium chloride filtered and washed with water and thereafter the 900 part elutriate is concentrated to 300 parts by evaporation of 600 parts water.

The solution is mixed with 5 parts standard nickel catalyst placed in an autoclave and hydrogenated at 150°C at 1810 psig. The hydrogenate is filtered to remove catalyst treated with mixed-bed ion exchange resin (40% strong cationic and 60% anionic) and concentrated. 174 parts product is produced containing 86 parts solid which consists of 49% sorbitol, 51% mannitol and minor amounts of iditol, hexitans, and other carbohydrates as determined by gas-liquid chromatographic analysis.

### EXAMPLE 2

A 400 part glucose epimerizate of Preparation I containing 51.6% solids is heated to 70°C and mixed with 80 grams sodium chloride. 100 parts water is removed from the solution under circulating air evaporation at 70°C. The solution is cooled and 50 parts precipitate is collected by filtration after which the filtrate is further concentrated overnight to remove a further 116 parts precipitate which is filtered to yield 167 parts filtrate containing 62% solids. The solids in the filtrate has an analysis of 23.8% sodium chloride, 38% mannose and 38% glucose.

### EXAMPLE 3

400 Parts glucose epimerizate containing 51.6% solids is heated to 70°C and mixed with 80 parts sodium chloride. 100 parts water is removed by evaporation and the solution is permitted to stand for 72 hours at room temperature. The precipitate is removed and has a glucose to mannose weight ratio of 92/8. The filtrate has a glucose/mannose ratio of 39/61 and contains minor amounts of fructose and disaccharides.

### EXAMPLE 4

1067 Parts purified epimerized glucose solution containing 726 parts carbohydrate solids of which 194 parts is mannose and 503 parts is glucose is heated to 70°C and intermixed with 104 parts sodium chloride with slow agitation. This solution is cooled slowly from 70° to 30°C over a period of 2 hours and thereafter cooled to 18 - 20°C over a period of 1/2 hour. The solution is permitted to stand at 18 - 20°C for a period of 5 hours and thereafter at room temperature (approximately 25°C) for an additional 5 hour period. The slurry is subjected to filtration under vacuum at room temperature. 439 parts filter cake containing 9.1% water and 5.2 sodium chloride is collected. On a dry basis, the filter cake contains 3.8% mannose (15 parts) and 87.7% glucose (350 parts), and 34 parts sodium chloride. The filtrate contains 32.8% water, 10.4% sodium chloride and on a dry basis 40.4% mannose (175 parts), 42.2% (183 parts) glucose and 15.5% (67 parts) sodium chloride. This analysis indicates that 90% of the available mannose remains with the filtrate along with 36% of the available glucose in the starting material.

The filtrate is passed through an exchange column to remove salt as described in Example 1 to yield on a dry basis a composition containing 47.6% mannose and 52.1% glucose. Upon hydrogenation as described in Example 1, a product containing mannitol and sorbitol in a similar amount is produced.

The filter cake is redissolved in water and recycled to the epimerization process as described in Preparation 1 wherein the mannose concentration is taken from 3.8% to as high as about 33%. The presence of sodium chloride appears to have no significant effect on the epimerization process.

### EXAMPLES 5 - 12

Employing starting materials and procedures similar to those shown for Examples 1 through 4 but with varying amounts of sodium halide expressed as parts sodium halide per 100 parts of
solid carbohydrate in the mannose/glucose epimerizate it has
been found that by employing about 18 to 20 parts sodium chloride
per 100 parts carbohydrate in the epimerizate solution the mannose
concentration in the filtrate is maximized when the solids content
of the epimerizate ranges from about 65 to 72 percent and preferably about 68 to 70 percent by weight. As indicated in Table 1
improved results are obtained when the mol ratio of sodium chloride
to carbohydrate sugar ranges from about .5 - .6 while lower ratios
appear to produce less effective concentration of mannose in the
filtrate and higher ratios appear to offer no substantial improvement.

## EXAMPLE 13

To demonstrate that sodium iodide can be used in place
of sodium chloride the procedure of Example 1 can be performed
employing 58 parts sodium iodide per 100 parts sugar to produce
a filtrate containing 49% mannose and a precipitate containing 10%
mannose.

## EXAMPLE 14

To demonstrate that sodium bromide can be used in place
of sodium chloride according to a procedure of Example 1,40 parts
sodium bromide per 100 parts sugar in the epimerizate can be
employed to produce a filtrate containing 58% mannose and a
precipitate containing 9% mannose. Results obtained for Examples
13 and 14 are listed in Table 1 below.

Table 1

| Example No | Parts Halide per 100 parts Carbohydrate | Mol Ratio Halide/ Carbohydrate | % Mannose in Filtrate | % Mannose in Precipitate |
|---|---|---|---|---|
| 5 | 9.1 (NaCl) | 0.28 | 35.4 | None detected |
| 6 | 18.2 (NaCl) | 0.56 | 56.0 | 4 |
| 7 | 18.2 (NaCl) | 0.56 | 61.0 | 8.6 |
| 8 | 18.2 (NaCl) | 0.56 | 51.5 | 5.3 |
| 9 | 18.2 (NaCl) | 0.56 | 55.6 | 9.0 |
| 9a* | – (NaCl) | – | 68.3 | None detected |
| 10 | 18.2 (NaCl) | 0.56 | 51.0 | None detected |
| 11 | 38.8 (NaCl) | 1.2 | 50.0 | None detected |
| 12 | 45.3 (NaCl) | 1.4 | 53.4 | None detected |
| 12a* | – (NaCl) | – | 72.0 | None detected |
| 13 | 58 (NaI) | .48 | 49 | 10 |
| 13a* | – (NaI) | – | 63 | 6.7 |
| 14 | 40 (NaBa) | .23 | 58 | 9.0 |

*   Filtrate after standing for 1 week to allow further precipitation to take place.

It is contemplated that the process of the invention can be carried out on a continuous or semi-continuous basis employing conventional process equipment, for example, glucose solution can be epimerized on a continuous basis by passing it through an ion exchange column having deposited thereon a molybdic acid catalyst. The epimerzate containing about 30% mannose and 60 – 88% carbohydrate can then be treated with sodium halide to form at least a saturated sodium halide solution and preferably a solution wherein the molar ratio of sodium halide to sugar ranges from about .5 to .6. If needed this solution can be passed into a concentrator to reduce the carbohydrate solids concentration to about 68 – 72% and thereafter a cooled precipitation to remove a precipitate employing a drum type filter or similar type filtration equipment on a continuous basis. The filtrate can be passed through an ion exchange column to remove sodium halide. The mannose rich elutreant can thereafter be hydrogenated in a pressure vessel employing a nickel catalyst to form a mannitol rich solution. The precipitate containing sodium halide salt of glucose can thereafter be redissolved and recycled along with additional glucose solution into the epimerization column. Pure mannitol can be more effectively recovered in higher concentrations employing a conventional crystalization technique to separate the mannitol from sorbitol in the mannitol rich solution.

## EXAMPLE 15

Three parts commercially available 95% glucose solution resulting from cornstarch is mixed with 1.5 parts glucose hydrate/ sodium chloride adduct and dissolved in deionized water to form a solution containing 55% carbohydrate solids. This solution is passed continuously at 90°C over a mixed-bed ion exchange resin having deposited thereon 1% by weight molybdic acid. The holdup time in the ion exchange column is about three hours to gain the optimum amount of epimerization.

The effluent epimerizate is mixed with sufficient brine and passed through a flash evaporator at about 70°C to form a solution containing 18.2 parts NaCl/100 parts carbohydrate solids and a carbohydrate solids concentration of 68 - 72%. The concentrate is cooled to a temperature of 20°C in a mildly agitated precipitator and filtered in rotating drum type filtration equipment to remove glucose hydrate/sodium chloride adduct which is recycled into the epimerization column. The mannose rich filtrate at 20°C is then passed through a mixed-bed anion/cation exchange resin column or an electrodialysis unit containing cation permeable and anion permeable membranes to demineralize the mannose rich filtrate. The demineralized solution is then passed through a flash evaporator to form a solution containing 76% solids and thereafter hydrogenated in the presence of Nickel Catalyst in an autoclave at 140°C to form a solution containing on a dry basis 51% mannitol and 49% sorbitol.

PA/JNA/MP
26 July 1982

1.      A process for concentrating mannose in an aqueous carbohydrate solution containing glucose and mannose having a carbohydrate
solids content of 60 - 88% by weight which comprises the steps of:

     (a)     forming an insoluble glucose hydrate/sodium
          halide adduct in said carbohydrate solution,

     (b)     separating said insoluble adduct from said
          aqueous solution, and

     (c)     collecting the mannose rich solution resulting
          therefrom.

2.      A process according to Claim 1 wherein the solids concentration of the carbohydrate solution is 68 - 72 percent by weight.

3.      A process according to Claim 1 or Claim 2 wherein the
sodium halide is sodium chloride, sodium iodide or sodium bromide.

4.      A process according to any one of the preceding claims
wherein Step b is carried out at a temperature of 20 - 70°C.

5.      A process according to Claim 3 wherein the sodium halide
is sodium chloride.

6.      A process according to Claim 5 wherein the sodium chloride/
glucose adduct has a composition wherein the ratio of glucose/mannose
is at least 20/1.

7.      A process according to any one of the preceding claims
wherein the mannose rich solution of Step c contains mannose and
glucose in a weight ratio range of at least 1/1 to 2.5/1 mannose/
glucose.

8.      A process for the production of mannitol from a carbohydrate solution containing glucose which comprises the steps of:

     (1)     epimerizing said carbohydrate solution containing
          glucose and mannose,

     (2)     concentrating mannose by the process of Claim 1
          to form an aqueous solution of mannose and
          glucose which is rich in mannose,

     (3)     demineralizing said mannose rich solution to
          remove sodium halide, and

     (4)     subjecting said demineralized solution to hydrogenation to form a solution rich in mannitol.

9.        A process according to Claim 8 wherein Step 2 is carried
out in a solution containing 68 - 72% carbohydrate solids.

10.       A process according to Claim 8 or Claim 9 wherein the
insoluble sodium halide/glucose hydrate adduct of Step 2 is recycled to Step 1.

11.       A process according to any one of Claims 8 to 10 wherein
the demineralization of Step 3 is carried out in a mixed-bed cation/
anion exchange column or in an electrodialysis unit.

12.       A process according to any one of Claims 8 to 11 wherein
said epimerization of Step 1 is carried out in a mixed-bed ion exchange column having deposited thereon a molybdic acid catalyst.

**European Patent Office**

## EUROPEAN SEARCH REPORT

## DOCUMENTS CONSIDERED TO BE RELEVANT

| Category | Citation of document with indication. where appropriate, of relevant passages | Relevant to claim | CLASSIFICATION OF THE APPLICATION (Int. Cl. ³) |
|---|---|---|---|
| | --- | | C 13 K 13/00 |
| Y | US-A-3 671 316 (RYOK TATUKI) *Column 3, lines 10-25; claims* | 1-12 | C 07 C 29/10 |
| | --- | | |
| Y | GB-A-1 540 556 (ICI AMERICAS) *Page 2, lines 19-36; claims* | 1-12 | |
| | --- | | |
| Y | EP-A-0 004 720 (I.C.I. AMERICAS) *Page 3, line 20* | 1,12 | |
| | --- | | |
| X | CHEMICAL ABSTRACTS, vol. 60, no. 10, 11th May 1964, no. 11423e, Columbus Ohio (USA); N.V.LEBEDEV et al.: "Equilibrium state of the glucose-NaCl-water system in the presence of mannose and xylose". & SB. TR., GOS. NAUCHN.-ISSLED. INST. GIDROLIZN. I SUL'FITNO-SPIRT. PROM. 9, 70-80(1961). *Abstract* | 1 | |
| | ----- | | **TECHNICAL FIELDS SEARCHED (Int. Cl ³)** |
| | | | C 13 K 3/00 |
| | | | C 13 K 13/00 |

The present search report has been drawn up for all claims

| Place of search THE HAGUE | Date of completion of the search 22-12-1982 | Examiner LENSEN H.W.M. |
|---|---|---|

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03 82